# DEMANDE DE BREVET EUROPEEN

(11) **EP 2 145 871 A1**
(43) Date de publication de la demande: **20.01.2010**
(21) Numéro de dépôt: 09290568.6
(22) Date de dépôt: 16.07.2009
(51) Int. Cl.: C07B 37/10, C07C 69/757, C07C 69/753, C07C 69/76, C07C 67/317, C07C 67/32, C07C 51/38, C07C 62/34, C07C 213/02, C07C 213/10, C07C 217/58, C07C 231/02, C07C 235/40, C07C 253/30, C07C 255/47, C07F 7/18, C07D 223/16

(54) **Nouveau procédé de préparation de benzocyclobutènes fonctionnalisés, et application à la synthèse de l'ivabradine et de ses sels d'addition à un acide pharmaceutiquement acceptable.**

(30) Priorité: 17.07.2008 FR 0804061
(71) Demandeur: Les Laboratoires Servier, 92284 Suresnes Cedex (FR); CNRS (Centre National de La Recherche Scientifique), 75794 Paris Cedex 16 (FR); Universite Claude Bernard Lyon 1, 69622 Villeurbanne Cedex (FR)
(72) Inventeur: Peglion, Jean-Louis, 78110 Le Vesinet (FR); Baudoin, Olivier, 69330 Meyzieu (FR); Audic, Nicolas, 80090 Amiens (FR); Chaumontet, Manon, 31200 Toulouse (FR); Piccardi, Riccardo, 92160 Antony (FR)
(74) Mandataire: Giudicelli, Cathy

(57) **Abrégé**

Procédé de préparation des composés de formule (IV) : 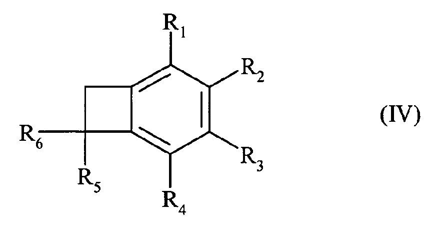 dans laquelle R₁, R₂, R₃ et R₄, identiques ou différents, représentent chacun un atome d'hydrogène, un groupement alkyle (C₁-C₆) linéaire ou ramifié, un groupement alkoxy (C₁-C₆) linéaire ou ramifié, un atome de fluor, de chlore, un groupement amine protégé, un groupement hydroxyle protégé, un groupement alkoxycarbonyle dans lequel le groupement alkoxy est (C₁-C₆) linéaire ou ramifié, un groupement CF₃, ou R₁=R₄=H et R₂ et R₃ forment ensemble avec les atomes de carbone qui les portent un groupement 1,3-dioxolane, R₅ représente un groupement alkyle (C₁-C₆) linéaire ou ramifié, saturé ou insaturé, un groupement hydroxyalkyle (C₁-C₆) linéaire ou ramifié dont la fonction hydroxyle est protégée, ou un groupement CO₂R₇ dans lequel R₇ est un groupement alkyle (C₁-C₆) linéaire ou ramifié,
R₆ représente un groupement cyano ou un groupement CO₂R₈ dans lequel R₈ est un groupement alkyle (C₁-C₆) linéaire ou ramifié.

Application à la synthèse de l'ivabradine, de ses sels d'addition à un acide pharmaceutiquement acceptable et de leurs hydrates.

## Description

La présente invention concerne un procédé de préparation de benzocyclobutènes fonctionnalisés, et leur application à la synthèse de l'ivabradine, de ses sels d'addition à un acide pharmaceutiquement acceptable et de leurs hydrates.

L'ivabradine de formule (I) : 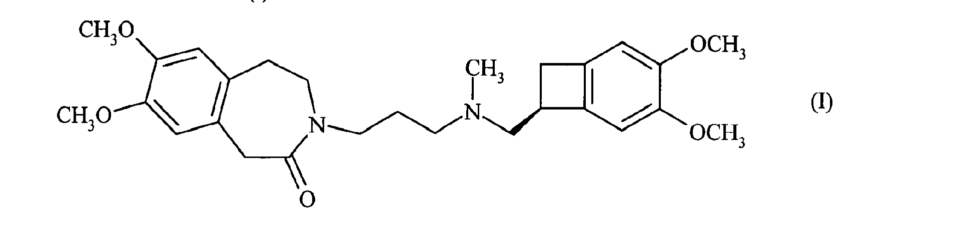 ou 3-{3-[{[(7*S*)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

Ce brevet décrit la synthèse de l'ivabradine à partir du composé de formule (II) :

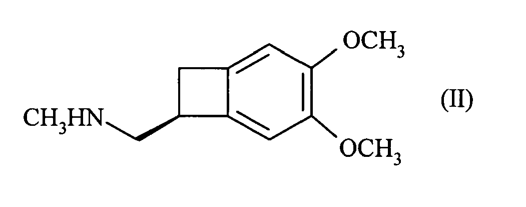

Le composé de formule (II) est obtenu par dédoublement, à l'aide d'acide camphosulfonique, du composé de formule (III):

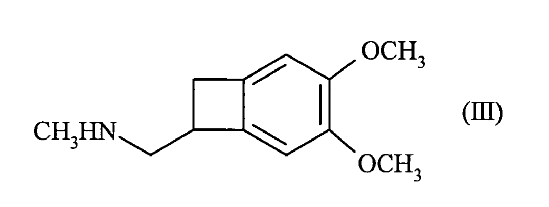

Le composé de formule (II) est un intermédiaire important dans la synthèse de l'ivabradine.

La présente invention concerne un procédé de préparation de benzocyclobutènes fonctionnalisés et leur application à la synthèse de l'ivabradine, de ses sels d'addition à un acide pharmaceutiquement acceptable et de leurs hydrates *via* le composé de formule (II).

La préparation de benzocyclobutènes fonctionnalisés a été décrite dans Angewandte Chemie, International Edition 2003, 42, 5736-5740. Cette publication décrit la possibilité d'appliquer l'activation de liaison C(sp³)-H à la préparation de benzocyclobutènes fonctionnalisés en utilisant un système catalytique au palladium.

Plus spécifiquement, la présente invention concerne un procédé de préparation des composés de formule (IV) : 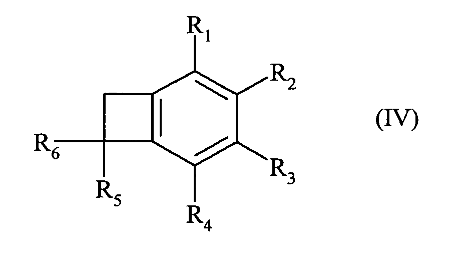 dans laquelle R₁, R₂, R₃ et R₄, identiques ou différents, représentent chacun un atome d'hydrogène, un groupement alkyle (C₁-C₆) linéaire ou ramifié, un groupement alkoxy (C₁-C₆) linéaire ou ramifié, un atome de fluor, de chlore, un groupement amine protégé, un groupement hydroxyle protégé, un groupement alkoxycarbonyle dans lequel le groupement alkoxy est (C₁-C₆) linéaire ou ramifié, un groupement CF₃, ou R₁=R₄=H et R₂ et R₃ forment ensemble avec les atomes de carbone qui les portent un groupement 1,3-dioxolane, R₅ représente un groupement alkyle (C₁-C₆) linéaire ou ramifié, saturé ou insaturé, un groupement hydroxyalkyle (C₁-C₆) linéaire ou ramifié dont la fonction hydroxyle est protégée, ou un groupement CO₂R₇ dans lequel R₇ est un groupement alkyle (C₁-C₆) linéaire ou ramifié,
R₆ représente un groupement cyano ou un groupement CO₂R₈ dans lequel R₈ est un groupement alkyle (C₁-C₆) linéaire ou ramifié,
**caractérisé en ce que** l'on soumet le composé de formule (V) : 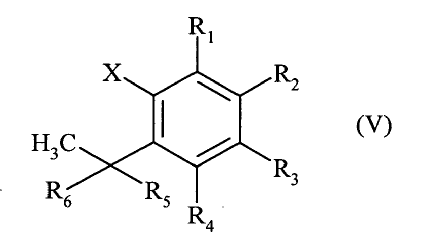 dans laquelle R₁, R₂, R₃, R₄, R₅, R₆ sont tels que définis précédemment et X représente un atome d'halogène, préférentiellement un atome de brome, ou un groupement triflate,
à une réaction de cyclisation en présence d'un système catalyseur/ligand comprenant un catalyseur au palladium et une phosphine organique, choisie parmi tri-*tert*-butyl phosphine, la 2-biphényl-di-*tert*-butylphosphine, le 1,2,3,4,5-pentaphényl-1'-(di-*tert-*butylphosphino)ferrocène et la tris(4-méthoxy-2-méthylphényl)phosphine, ou un sel de phosphonium desdites phosphines,
d'une base,
dans un solvant organique.

Par groupement hydroxyle protégé ou fonction hydroxyle protégée, on entend une fonction hydroxyle substituée par un groupement protecteur usuel de cette fonction. Parmi ces groupements protecteurs, on peut citer à titre non limitatif les groupements silylés comme le triisopropylsilyle, le *tert*-butyldiméthylsilyle, les groupements tétrahydropyrane, benzyle, para-méthoxybenzyle, trityle, acétyle et pivaloyle.

Par groupement amine protégé, on entend une fonction amine substituée par un groupement protecteur usuel de cette fonction. Parmi ces groupements protecteurs, on peut citer à titre non limitatif les groupements nosyle, tosyle, mésyle, acétyle, *tert-*butoxycarbonyle, benzyle et phtalimide.

Parmi les catalyseurs au palladium pouvant être utilisés on peut citer à titre non limitatif Pd(OAc)₂, Pd(dba)₂, Pd₂(dba)₃, PdCl₂, PdCl₂(CH₃CN)₂, PdBr₂, ou le *trans*-di-(µ-acétate)bis[o-(di-o-tolylphosphine)benzyl]dipalladium (catalyseur d'Herrmann).

Le catalyseur au palladium préférentiellement utilisé est Pd(OAc)₂.

La phosphine organique préférentiellement utilisée est la tri-*tert*-butyl phosphine.

Parmi les sels de phosphonium pouvant être utilisés on peut citer à titre non limitatif les tétrafluoroborates, les hexafluorophosphates et les hexafluoroantimonates de phosphonium.

Le sel de phosphonium préférentiellement utilisé est le tétrafluoroborate de *tri*-*tert-*butylphosphonium.

Parmi les bases pouvant être utilisées on peut citer à titre non limitatif K₂CO₃, CS₂CO₃, Na₂CO₃, K₃PO₄, KHCO₃, *t*-BuCO₂Na, *t*-BuCO₂K, *t*-BuCO₂Cs.

La base préférentiellement utilisée est K₂CO₃.

Parmi les solvants pouvant être utilisés on peut citer à titre non limitatif le DMF, le *N,N-*diméthylacétamide, la *N*-méthylpyrrolidine, le xylène,le mésitylène.

Le solvant préférentiellement utilisé est le DMF.

La température de la réaction est préférentiellement comprise entre 100°C et 150°C.

Selon un mode de réalisation préféré, la présente invention concerne le procédé de préparation des composés de formule (IVa), cas particuliers des composés de formule (IV) pour lesquels R₅=CO₂R₇ et R₆=CO₂R₈ : 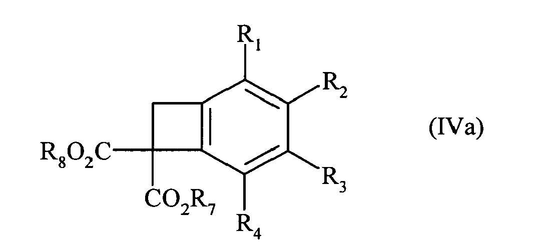 dans laquelle R₁, R₂, R₃, R₄, R₇ et R₈ sont tels que définis précédemment,
à partir des composés de formule (Va), cas particuliers des composés de formule (V) pour lesquels R₅=CO₂R₇ et R₆=CO₂R₈ : 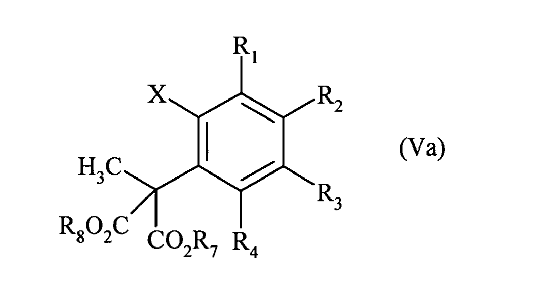 dans laquelle R₁, R₂, R₃ R₄, R₇, R₈ et X sont tels que définis précédemment.

Selon un autre mode de réalisation préféré, la présente invention concerne un procédé de préparation des composés de formule (IVb), cas particuliers des composés de formule (IVa) pour lesquels R₁=R₄=H et R₂=R₃=OCH₃ : 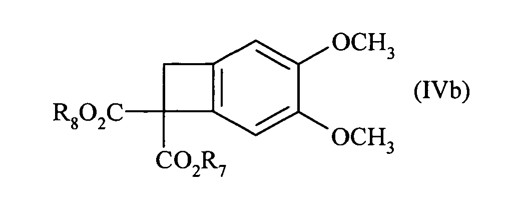 dans laquelle R₇ et R₈ sont tels que définis précédemment,
à partir des composés de formule (Vb), cas particuliers des composés de formule (Va) pour lesquels R₁=R₄=H et R₂=R₃=OCH₃ : 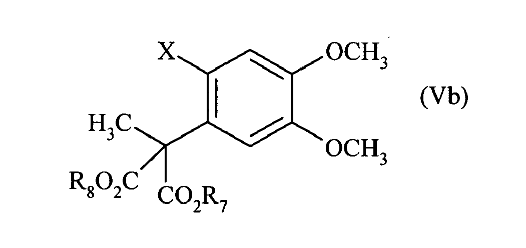 dans laquelle R₇, R₈ et X sont tels que définis précédemment.

Les composés de formule (IVa), obtenus selon le procédé de l'invention, peuvent conduire aux composés de formule (VIa) : 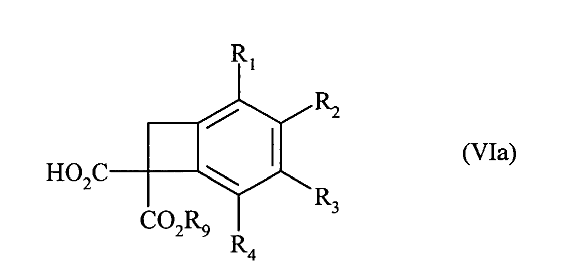 dans laquelle R₁, R₂, R₃ et R₄ sont tels que définis précédemment,
et R₉ est un groupement alkyle (C₁-C₆) linéaire ou ramifié,
par une réaction d'hydrolyse ou de saponification d'ester,
puis les composés de formule (VIa) conduisent aux composés de formule (VIIa) : 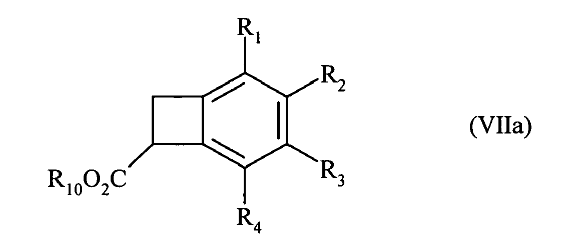 dans laquelle R₁, R₂, R₃ et R₄ sont tels que définis précédemment,
et R₁₀ est un atome d'hydrogène ou un groupement alkyle (C₁-C₆) linéaire ou ramifié, par une réaction de décarboxylation.

L'hydrolyse ou la saponification d'une fonction ester des composés de formule (IVb) conduit aux composés de formule (VIb), cas particuliers des composés de formule (VIa) pour lesquels R₁=R₄=H et R₂=R₃=OCH₃ : 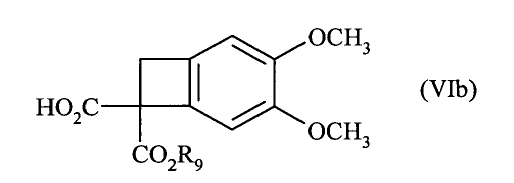 dans laquelle R₉ est tel que défini précédemment.

Puis la décarboxylation des composés de formule (VIb) conduit aux composés de formule (VIIb), cas particuliers des composés de formule (VIIa) pour lesquels R₁=R₄=H et R₂=R₃=OCH₃ : 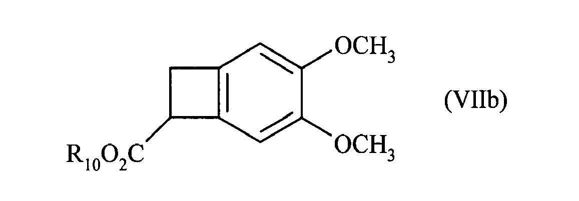 dans laquelle R₁₀ est tel que défini précédemment.

Les composés de formule (VIIb) obtenus selon le procédé de l'invention sont utiles dans la synthèse de l'ivabradine, de ses sels d'addition à un acide pharmaceutiquement acceptable et de leurs hydrates.

A titre d'exemple, leur réaction avec la méthylamine conduit au composé de formule (VIII) : 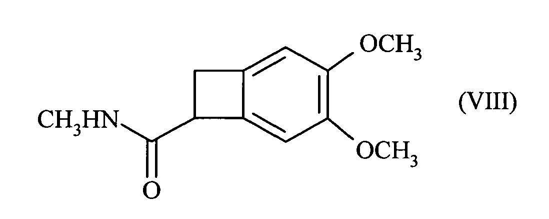 dont la réduction conduit au composé de formule (III): 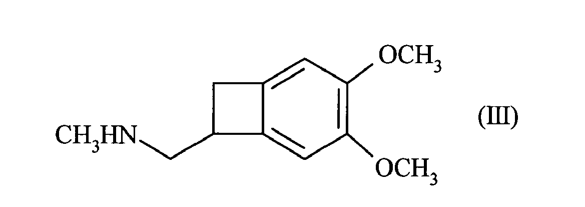 dont le dédoublement à l'aide d'acide camphosulfonique conduit au composé de formule (II) : 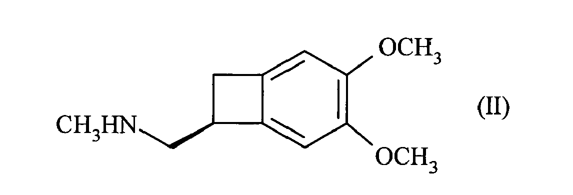 qui est transformé en ivabradine de formule (I) 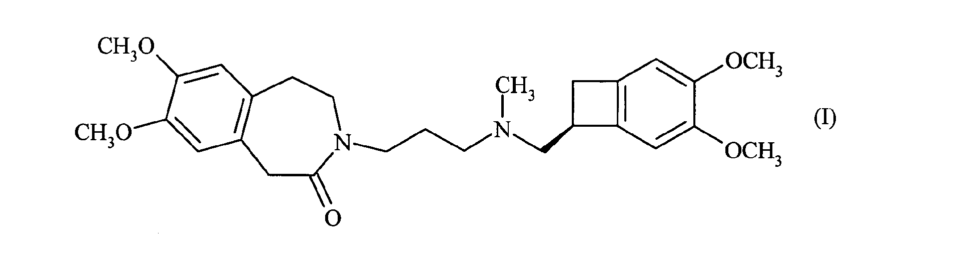 ou 3-{3-[{[(7*S*)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Parmi les méthodes connues pour effectuer la transformation du composé de formule (II) en ivabradine, on peut citer celles décrites dans les brevets européens EP 0 534 859 et EP 1 589 005.

L'obtention des dérivés de formule (IVb) avec de bons rendements est donc particulièrement utile dans la synthèse de l'ivabradine, de ses sels d'addition à un acide pharmaceutiquement acceptable et de leurs hydrates.

Les composés de formule (IVa), cas particuliers des composés de formule (IV) pour lesquels R₅=CO₂R₇ et R₆=CO₂R₈, ainsi que les composés de formule (VIb), et les composés de formule (VIIc), cas particuliers des composés de formules (VIIb) pour lesquels R₁₀ représente un groupement alkyle (C₁-C₆) linéaire ou ramifié, sont des produits nouveaux, utiles comme intermédiaires de synthèse dans l'industrie chimique ou pharmaceutique, notamment dans la synthèse de l'ivabradine, de ses sels d'addition à un acide pharmaceutiquement acceptable et de leurs hydrates, et font à ce titre partie intégrante de la présente invention.

### Liste des abréviations utilisées :

dba : dibenzylidène acétone
DMF : *N*,*N*-diméthylformamide
DMSO : diméthylsulfoxyde
éq. : équivalent
THF : tétrahydrofurane

Les exemples ci-dessous illustrent l'invention.

### Procédure générale A de formation de benzocyclobutènes par activation de liaison C-H

Charger, dans un tube de Schlenk sec et refermable hermétiquement, équipé d'un barreau aimanté, le bromure arylique (1 mmol), Pd(OAc)₂ (0.1 éq.), P(*t*-Bu)₃.HBF₄ (0,2 éq.) et K₂CO₃ anhydre (1,3 éq.). Le tube de Schlenk est purgé et placé sous argon. 4 mL de DMF anhydre sont ajoutés sous argon, le tube de Schlenk est fermé hermétiquement puis mis sous agitation dans un bain d'huile préchauffé à 140°C jusqu'à disparition complète du bromure arylique en GC/MS. Après retour à température ambiante, le milieu réactionnel est dilué dans le diéthyléther et filtré sur Célite^{®}. La solution organique est lavée par une solution aqueuse saturée de NaCl, séchée sur MgSO₄ et le solvant résiduel est évaporé sous pression réduite. Le brut réactionnel est purifié par chromatographie flash pour conduire au benzocyclobutène.

### Procédure générale B de formation de benzocyclobutènes par activation de liaison CH

Charger, dans un tube de Schlenk sec et refermable hermétiquement, équipé d'un barreau aimanté, le bromure arylique (1 mmol), Pd₂(dba)₃ (0,05 éq.), P(*t*-Bu)₃.HBF₄ (0,1 éq.) et K₂CO₃ anhydre (1,3 éq.). Le tube de Schlenk est purgé et placé sous argon. 4 mL de DMF anhydre sont ajoutés sous argon, le tube de Schlenk est fermé hermétiquement puis mis sous agitation dans un bain d'huile préchauffé à 120°C jusqu'à disparition complète du bromure arylique en GC/MS. Après retour à température ambiante, le milieu réactionnel est dilué dans le diéthyléther et filtré sur Célite^{®}. La solution organique est extraite par une solution aqueuse saturée de NaCl, séchée sur MgSO₄ et le solvant résiduel est évaporé sous pression réduite. Le brut réactionnel est purifié par chromatographie flash pour conduire au benzocyclobutène.

### Exemple 1 : 7-Méthylbicyclo[4.2.0]octa-1,3,5-triène-3,7-dicarboxylate de diméthyle

Suivant la procédure générale A à partir du 3-bromo-4-(2-méthoxy-1,1-diméthyl-2-oxoéthyl)benzoate de méthyle, le composé du titre est obtenu avec 92% de rendement.
**IR (film) :** ν= 2952, 1714, 1433, 1275, 1146, 1079, 768 cm⁻¹.

### Exemple 2 : 7-Méthyl-4-(trifluorométhyl)bicyclo[4.2.0]octa-1,3,5-triène-7-carboxylate de méthyle

Suivant la procédure générale A à partir du 2-[2-bromo-5-(trifluorométhyl)phényl]-2-méthylpropanoate de méthyle, le composé du titre est obtenu avec 81 % de rendement.
**IR (film) :** ν= 2955, 1731,1315, 1143, 1113, 826, 685 cm⁻¹.

### Exemple 3 : 7-isopropylbicyclo[4.2.0]octa-1,3,5-triène-7-carbonitrile

Suivant la procédure générale A à partir du 2-(2-bromophényl)-2,3-diméthylbutanenitrile, le composé du titre est obtenu avec 72% de rendement.
**IR (film) :** ν= 2964, 1458, 748, 715 cm⁻¹.

### Exemple 4 : 3-Méthylbicyclo[4.2.0]octa-1,3,5-triène-7,7-dicarboxylate de diméthyle

Suivant la procédure générale A à partir du 2-(2-bromo-4-méthylphényl)-2-méthylmalonate de diméthyle, le composé du titre est obtenu avec 87% de rendement.
**IR (film)** : ν= 2952, 1731 cm⁻¹.

### Exemple 5 : 3,4-Diméthoxybicyclo[4.2.0]octa-1,3,5-triène-7,7-dicarboxylate de diméthyle

Le composé du titre est obtenu en suivant la procédure générale A à partir du 2-(2-bromo-4,5-diméthoxyphényl)-2-méthylmalonate de diméthyle.
**IR (pur)** : ν= 3007, 2960, 1730, 1591, 1239, 1116, 1090, 878, 758 cm⁻¹.

### Exemple 6 : 3,4-Diméthoxybicyclo[4.2.0]octa-1,3,5-triène-7,7-dicarboxylate de tert-butyle et de méthyle

Suivant la procédure générale A à partir de 2-(2-bromo-4,5-diméthoxyphényl)-2-méthylmalonate de *tert*-butyle et de méthyle, le composé du titre est obtenu avec 69% de rendement.
**IR (pur)** : ν= 2977, 2936, 1728, 1591, 1464, 1249, 1115, 840 cm⁻¹.

### Exemple 7 : 7-{4-[(Triisopropylsilyl)oxy]butyl}bicyclo[4.2.0]octa-1,3,5-triène-7-carboxylate de méthyle

Suivant la procédure générale A à partir du 2-(2-bromophényl)-2-méthyl-6-[(triisopropylsilyl)oxy]hexanoate de méthyle, le composé du titre est obtenu avec 82% de rendement.
**IR (film) :** ν= 2940, 2863, 1731, 1457, 1253, 1103, 881, 678 cm⁻¹.

### Exemple 8 : 7-Méthylbicyclo[4.2.0]octa-1,3,5-triène-7-carboxylate de méthyle

Suivant la procédure générale B à partir du 2-(2-bromophényl)-2-méthylpropanoate de méthyle, le composé du titre est obtenu avec 81 % de rendement.
**IR (pur) :** ν= 2972, 2952, 2930, 1729, 1457, 1433, 1277, 1140, 740, 711 cm⁻¹.

### Exemple 9 : Acide (R,S)-3,4-diméthoxybicyclo[4.2.0]octa-1,3,5-triène-7-carboxylique

638 mg (2,28 mmol)de 3,4-diméthoxybicyclo[4.2.0]octa-1,3,5-triène-7,7-dicarboxylate de diméthyle obtenu à l'exemple 5 sont dissous dans 18 mL de DMSO et 445 mg (6,84 mmol) de KCN sont ajoutés en une fois. Le mélange est agité sous atmosphère d'argon à 130°C pendant 12h. Après retour à température ambiante, 25 mL de solution aqueuse 1N d'HCl et 20 mL de diéthyléther sont ajoutés avec précaution au mélange réactionnel **(ATTENTION :** formation d'HCN) et le mélange est agité 1h. La phase organique est lavée avec un solution aqueuse 1N de NaOH (3x10 mL). Les phases aqueuse sont réunies puis acidifiées à pH 2 avec une solution aqueuse 6N d'HCl à température ambiante et extraites par le diéthyléther (3 x 15 mL). Les phases organiques sont séchées sur MgSO₄ et les solvants résiduels évaporés sous pression réduite. Le brut réactionnel est purifié par chromatographie flash (hexane / acétate d'éthyle : 85/15) pour conduire au composé du titre.
**IR (pur) :** ν= 3227, 2842, 2835, 1725, 1593, 1486, 1302, 1160, 1140, 970, 753 cm⁻¹.

### Exemple 10: (R,S)-3,4-Diméthoxybicyclo[4.2.0]octa-1,3,5-triène-7-carboxylate de méthyle

424 mg (1,31 mmol)de 3,4-Diméthoxybicyclo[4.2.0]octa-1,3,5-triène-7,7-dicarboxylate de *tert*-butyle et de méthyle obtenu à l'exemple 6 sont dissous dans 2,1 mL d'acide trifluoroacétique (99%) et le mélange est porté à reflux pendant 1,5h. Après retour à température ambiante le mélange est évaporé sous pression réduite pour conduire au mono acide carboxylique mono ester méthylique.

Le composé brut est dissous dans 4,34 mL d'un mélange 30 :1 DMF/pyridine et la solution est chauffée à 120°C sous atmosphère d'argon pendant 2h puis ramenée à température ambiante. Le mélange réactionnel est hydrolysé par une solution aqueuse saturée de NH₄Cl et la phase aqueuse est extraite avec de l'acétate d'éthyle (3 x 10 mL). Les phases organiques sont réunies, lavées par une solution aqueuse saturée de NaCl (20 mL), séchées sur MgSO4 et évaporées sous pression réduite. Le brut réactionnel est purifié par chromatographie flash (hexane/AcOEt : 8/2) pour conduire au produit du titre.
**IR (pur) :** ν= 2950, 2834, 1729, 1464, 1207, 1068, 729 cm⁻¹.

### Exemple 11 : (R,S) 3,4-diméthoxy N-méthylbicyclo[4.2.0] octa-1,3,5-triène 7-carboxamide

A une solution de 2,5 g (12 mmol) d'acide (*R*,*S*) 3,4-diméthoxybicyclo[4.2.0]octa-1,3,5-triène 7-carboxylique obtenu à l'exemple 9 dans 40 mL de méthanol, sont ajoutés 0,05 mL d'acide sulfurique (d = 1,83). Le mélange est chauffé au reflux pendant 2h puis refroidi à 10°C. 40 mL d'une solution à 40% de méthylamine dans l'eau sont ajoutés en 15 min et le mélange est laissé sous agitation pendant 2h. Le méthanol est évaporé sous pression réduite, 40mL d'eau sont ajoutés. Après extraction au CH₂Cl₂, les phases organiques réunies sont lavées successivement par de l'eau, de l'HCl 1N, et une solution saturée de NaCl puis séchées sur MgSO₄. Après évaporation des solvants sous pression réduite, 2,2g du produit du titre sont obtenus sous forme d'un solide beige (Rdt : 83%).
**Point de fusion :** 142-147°C

### Exemple 12 : Chlorhydrate de (R,S) 1-(3,4-diméthoxy bicyclo[4.2.0] octa-1,3,5-triène 7-yl) N-méthyl méthanamine

20 mL d'une solution molaire de BH₃ dans le THF sont ajoutés à température ambiante au mélange de 2,2 g (10 mmol) du produit obtenu à l'exemple 11 dans 45mL de THF. Après 1h sous agitation, 10mL de la solution de BH₃ dans le THF sont ajoutés. Après une nuit d'agitation à température ambiante, 20 mL d'éthanol sont additionnés goutte à goutte et le mélange est agité jusqu'à fin de dégagement gazeux (1h environ). 20 mL d'une solution d' HCl dans l'éthanol sont ensuite additionnés goutte à goutte. Après 4h sous agitation, le précipité obtenu (1,2 g de produit du titre) est filtré. Le filtrat est concentré et 0,65g supplémentaires de produit du titre sont obtenus par concrétisation dans un mélange AcOEt/éthanol 80 :20.

Les deux précipités sont réunis pour conduire à 1,85 g du produit du titre (Rdt 77%).
**Point de fusion :** 174-177°C

## Revendications

1. Procédé de préparation des composés de formule (IV) : 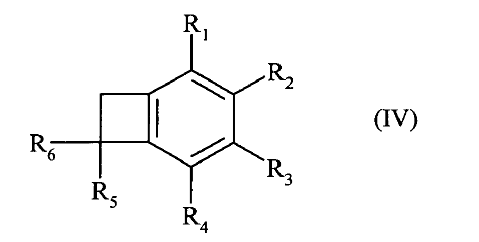 dans laquelle R₁, R₂, R₃ et R₄, identiques ou différents, représentent chacun un atome d'hydrogène, un groupement alkyle (C₁-C₆) linéaire ou ramifié, un groupement alkoxy (C₁-C₆) linéaire ou ramifié, un atome de fluor, de chlore, un groupement amine protégé, un groupement hydroxyle protégé, un groupement alkoxycarbonyle dans lequel le groupement alkoxy est (C₁-C₆) linéaire ou ramifié, un groupement CF₃, ou R₁=R₄=H et R₂ et R₃ forment ensemble avec les atomes de carbone qui les portent un groupement 1,3-dioxolane, R₅ représente un groupement alkyle (C₁-C₆) linéaire ou ramifié, saturé ou insaturé, un groupement hydroxyalkyle (C₁-C₆) linéaire ou ramifié dont la fonction hydroxyle est protégée, ou un groupement CO₂R₇ dans lequel R₇ est un groupement alkyle (C₁-C₆) linéaire ou ramifié,
R₆ représente un groupement cyano ou un groupement CO₂R₈ dans lequel R₈ est un groupement alkyle (C₁-C₆) linéaire ou ramifié,
**caractérisé en ce que** l'on soumet le composé de formule (V) 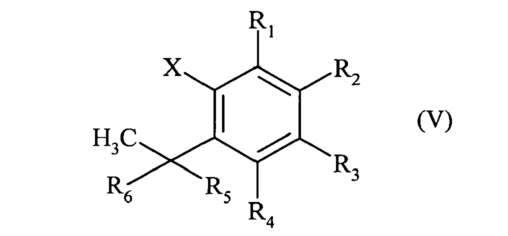 dans laquelle R₁, R₂, R₃, R₄, R₅, R₆ sont tels que définis précédemment et X représente un atome d'halogène, préférentiellement un atome de brome, ou un groupement triflate,
à une réaction de cyclisation en présence d'un système catalyseur/ligand comprenant un catalyseur au palladium et une phosphine organique, choisie parmi la tri-*tert*-butyl phosphine, la 2-biphényl-di-*tert*-butylphosphine, le 1,2,3,4,5-pentaphényl-1'-(di-*tert-*butylphosphino)ferrocène et la tris(4-méthoxy-2-méthylphényl)phosphine, ou un sel de phosphonium desdites phosphines,
d'une base,
dans un solvant organique.

2. Procédé de préparation selon la revendication 1, **caractérisé en ce que** le catalyseur au palladium est choisi parmi Pd(OAc)₂, Pd(dba)₂, Pd₂(dba)₃, PdCl₂, PdCl₂(CH₃CN)₂, PdBr₂, ou le *trans*-di-(µ-acétate)bis[*o*-(di-*o*-tolylphosphine)benzyl]dipalladium.

3. Procédé de préparation selon l'une quelconque des revendications 1 ou 2, **caractérisé en ce que** le catalyseur au palladium est Pd(OAc)₂.

4. Procédé de préparation selon l'une quelconque des revendications 1 à 3, **caractérisé en ce que** la phosphine organique est la tri-*tert*-butylphosphine.

5. Procédé de préparation selon l'une quelconque des revendications 1 à 4 **caractérisé en ce que** le sel de phosphonium est un tétrafluoroborate, un hexafluorophosphate ou un hexafluoroantimonate de phosphonium.

6. Procédé de préparation selon la revendication 5, **caractérisé en ce que** le sel de phosphonium est le tétrafluoroborate de tri-*tert*-butylphosphonium.

7. Procédé de préparation selon l'une quelconque des revendications 1 à 6, **caractérisé en ce que** la base utilisée est choisie parmi K₂CO₃, Cs₂CO₃, Na₂CO₃, K₃PO₄, KHCO₃, *t-*BuCO₂Na, *t*-BuCO₂K, *t*-BuCO₂Cs.

8. Procédé de préparation selon la revendication 7, **caractérisé en ce que** la base utilisée est K₂CO₃.

9. Procédé de préparation selon l'une quelconque des revendications 1 à 8, **caractérisé en ce que** le solvant organique utilisé est choisi parmi le DMF, le *N,N-*diméthylacétamide, la *N*-méthylpyrrolidine, le xylène, le mésitylène.

10. Procédé de préparation selon la revendication 9, **caractérisé en ce que** le solvant organique utilisé est le DMF.

11. Procédé de préparation selon l'une quelconque des revendications 1 à 10, **caractérisé en ce que** la température de la réaction est comprise entre 100°C et 150°C.

12. Procédé de préparation des composés de formule (VIIa) : 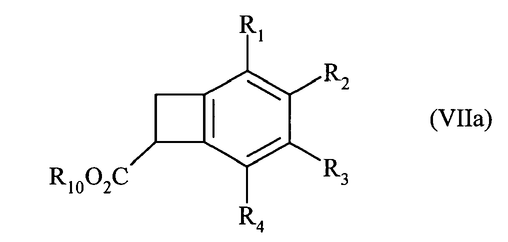 dans laquelle R₁, R₂, R₃ et R₄ sont tels que définis dans la revendication 1,
et R₁₀ est un atome d'hydrogène ou un groupement alkyle (C₁-C₆) linéaire ou ramifié, **caractérisé en ce que** le composé de formule (Va), cas particulier des composés de formule (V) pour lesquels R₅=CO₂R₇ et R₆=CO₂R₈ : 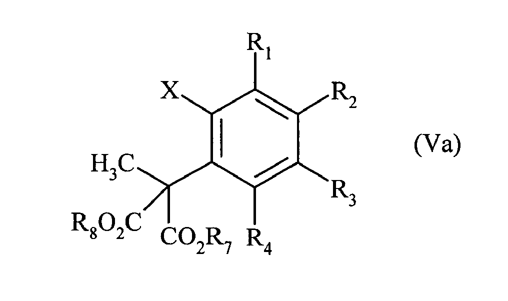 dans laquelle R₁, R₂, R₃, R₄, R₇, R₈ et X sont tels que définis dans la revendication 1,
est transformé en composé de formule (IVa), cas particulier des composés de formule (IV) pour lesquels R₅=CO₂R₇ et R₆=CO₂R₈ : 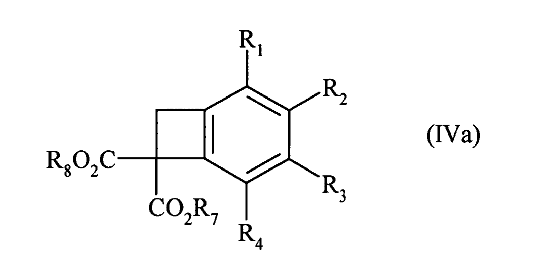 dans laquelle R₁, R₂, R₃, R₄, R₇ et R₈ sont tels que définis dans la revendication 1, selon le procédé de la revendication 1,
puis le composé de formule (IVa) est transformé en composé de formule (VIa) : 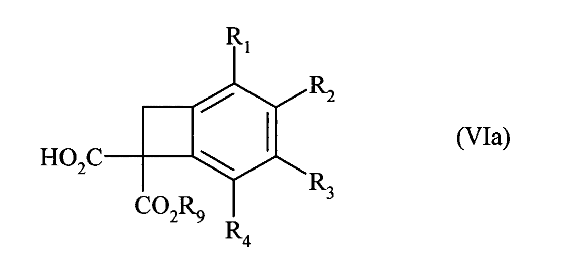 dans laquelle R₁, R₂, R₃ et R₄ sont tels que définis dans la revendication 1,
et R₉ est un groupement alkyle (C₁-C₆) linéaire ou ramifié,
par une réaction d'hydrolyse ou de saponification d'ester,
et le composé de formule (VIa) conduit au composé de formule (VIIa) par une réaction de décarboxylation.

13. Procédé de préparation selon la revendication 12 des composés de formule (VIIb), cas particulier des composés de formule (VIIa) pour lesquels R₁=R₄=H et R₂=R₃=OCH₃.

14. Composé de formule (IVa) : 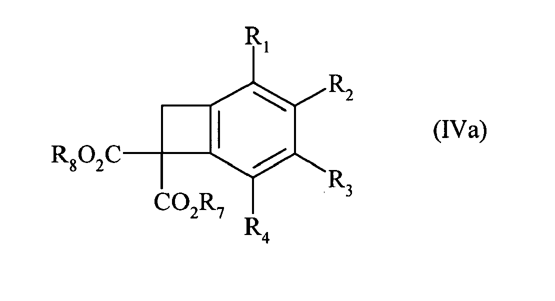 dans laquelle R₁, R₂, R₃ et R₄, identiques ou différents, représentent chacun un atome d'hydrogène, un groupement alkyle (C₁-C₆) linéaire ou ramifié, un groupement alkoxy (C₁-C₆) linéaire ou ramifié, un atome de fluor, de chlore, un groupement amine protégé, un groupement hydroxyle protégé, un groupement alkoxycarbonyle dans lequel le groupement alkoxy est (C₁-C₆) linéaire ou ramifié, un groupement CF₃, ou R₁=R₄=H et R₂ et R₃ forment ensemble avec les atomes de carbone qui les portent un groupement 1,3-dioxolane, et R₇ et R₈, identiques ou différents, représentent chacun un groupement alkyle (C₁-C₆) linéaire ou ramifié.

15. Composé de formule (VIb) : 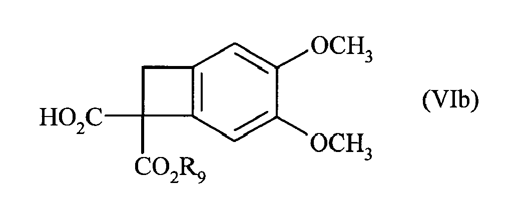 dans laquelle R₉ est un groupement alkyl (C₁-C₆) linéaire ou ramifié.

16. Composé de formule (VIIc): 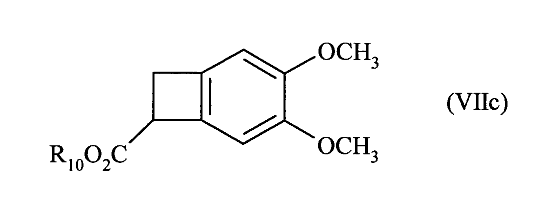 dans laquelle R₁₀ est un groupement alkyl (C₁-C₆) linéaire ou ramifié.

17. Procédé de synthèse de l'ivabradine, de ses sels pharmaceutiquement acceptables et de ses hydrates **caractérisé en ce que** le composé de formule (Vb) : 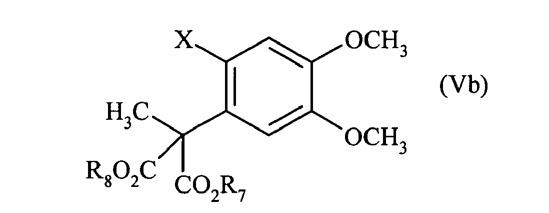 dans laquelle R₇, R₈ et X sont tels que définis dans la revendication 1,
est transformé en composé de formule (VIIb) suivant le procédé de la revendication 13 : 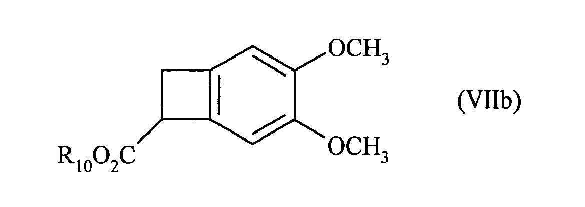 dans laquelle R₁₀ est tel que défini dans la revendication 12,
puis le composé de formule (VIIb) est transformé en composé de formule (VIII) : 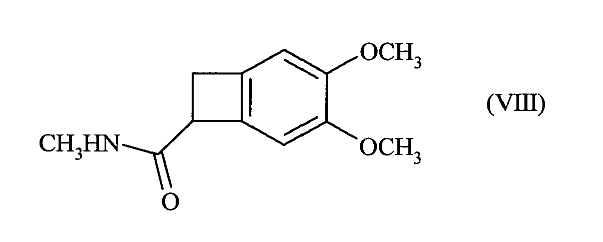 par réaction avec la méthylamine,
dont la réduction conduit au composé de formule (III): 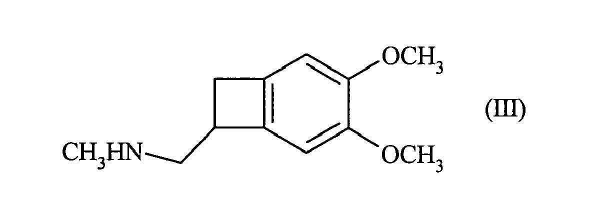 dont le dédoublement en présence d'acide camphosulfonique conduit au composé de formule (II) : 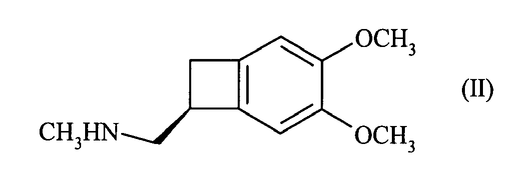 qui est transformé en ivabradine de formule (I) : 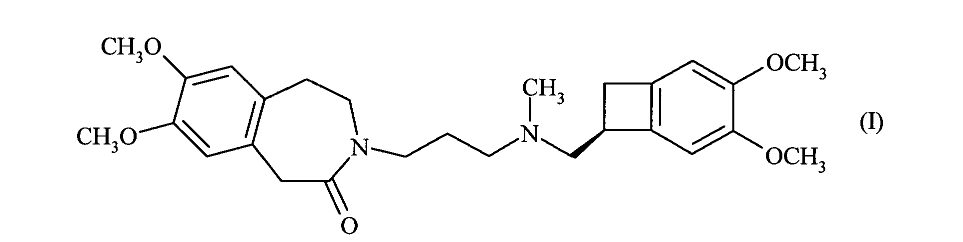 qui peut être transformée en ses sels d'addition à un acide pharmaceutiquement acceptable choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.
